(19) Europäisches Patentamt
European Patent Office
Office européen des brevets

(11) **EP 3 466 321 A1**

(12) **EUROPEAN PATENT APPLICATION**

(43) Date of publication:
**10.04.2019 Bulletin 2019/15**

(51) Int Cl.:
*A61B 5/00* (2006.01)    *A61B 5/05* (2006.01)

(21) Application number: **17195569.3**

(22) Date of filing: **09.10.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Micrima Limited
Bristol BS2 0EL (GB)**

(72) Inventor: **IRIARTE, Ana
Bristol BS2 0EL (GB)**

(74) Representative: **Haseltine Lake LLP
Redcliff Quay
120 Redcliff Street
Bristol BS1 6HU (GB)**

(54) **A BREAST DENSITY METER AND METHOD**

(57)    There is disclosed a breast density meter for measuring the density of breast tissue. The meter comprises at least one microwave antenna configured to transmit microwave signals so as to illuminate a breast of a patient and to receive the microwave signals following scattering within the breast; and a processor configured to generate intensity data based on the received microwave signals and determine a density indication or a cancer risk indication based on the intensity data and reference data.

FIG. 2

## Description

The invention relates to a breast density meter and method for measuring the density of breast tissue *in vivo.*

According to the World Health Organization, breast cancer is the second most common type of cancer and the fifth most common cause of cancer death. In view of the commonality of breast cancer, diligent individuals subject themselves to regular mammograms for the purpose of detecting an existence of breast cancer.

An ancillary benefit of having a mammogram conducted is the ability of the radiologist to determine a radiographical density of the participant's breast tissue due to the fact that there is a prognostic relationship between breast density and cancer risk. In general, it is known in the art that the radiographical density of a breast illustrated within a mammogram may vary due to differences in the amount of fat, connective tissues, and epithelial tissues that are present.

For example, because fibroglandular and connective tissues (i.e. glands, ducts, and fibers) have a relatively high x-ray attenuation, fibroglandular and connective tissues may appear to be radiographically dense/light on radiographic films. By contrast, fat has a relatively low x-ray attenuation and therefore appears to be the least radiographically dense/dark, when compared to the remaining breast tissue. Because of the distinct differences in x-ray attenuation between fat and fibroglandular tissue, segmentation of fibroglandular tissue from the rest of the breast is possible.

A known breast density estimation standard may be based upon a four-category Breast Imaging Reporting and Data Systems (BI-RADS) lexicon. Upon visually assessing a mammogram, a radiologist may classify the radiographical image of the breast into one of four BI-RADS compositional categories defined as:

> 1: Fatty - the breasts are almost entirely fatty;
> 2: Scattered - there are scattered areas of fibroglandular density;
> 3: Heterogeneous - the breasts are heterogeneously dense, which may obscure small masses; and
> 4: Dense - the breasts are extremely dense, which lowers the sensitivity of mammography.

Women whose breasts are categorized in the densest, fourth category are four-to-six times more likely to develop breast cancer than those categorized in the first, fatty category.

The above standard in breast density estimation involves a radiologist's visual assessment of a mammogram, and so is a subjective assessment which relies on the perception of the radiologist. While such a subjective density classification is quick to use and widely employed, it has been proven to be limited due to considerable intra- and inter-reader variability of a radiologist.

Accordingly, it may be desirable to provide an improved system and method for determining breast density.

There is disclosed a breast density meter for measuring the density of breast tissue comprising: at least one microwave antenna configured to transmit microwave signals over a range of frequencies so as to illuminate a breast of a patient and to receive the microwave signals following scattering within the breast; and a processor configured to generate intensity data based on the received microwave signals and determine a density indication or a cancer risk indication based on the intensity data and reference data. The intensity data may be related to image data. It may be possible to generate an image of the breast using the image data. The intensity data may be image pixel data or image voxel data.

The processor may be configured to determine a parameter value based on the intensity data; and may determine a density indication or a cancer risk indication based on a comparison between the parameter value and the reference data. The intensity data may comprise a set of plural intensity values determined based on the intensity (or magnitude) of the received microwave signals. The intensity values may be image pixel or image voxel intensity values.

The intensity data may comprise histogram data representing a frequency density distribution curve across a range of intensity values, the intensity values being determined based on the intensity (or magnitude) of the received microwave signals. The intensity values may be image pixel or image voxel intensity values. The histogram data may be image histogram data. The processor may be configured to determine the parameter value based on the histogram data.

The processor may be configured to determine the parameter value based on an area under at least a part of the curve that is represented by the histogram data. The processor may be configured to determine the parameter value based on a sum of elements with respect to at least a part of the curve that is represented by the histogram data.

The processor may be configured to: determine the parameter value for only a part of the frequency density distribution curve having intensity values falling within a range within the highest 50%, 45%, 40%, 35%, 30%, 25%, 20%, 15%, 10% or 5% of intensity values in the range of intensity values.

The reference data may comprise one or more reference values.

According to an aspect there is provided a method for measuring the density of breast tissue comprising: illuminating a breast of a patient with microwave signals emitted by at least one microwave antenna; receiving the microwave signals following scattering within the breast at said at least one microwave antenna; generating intensity data based on the received microwave signals; and determining a density indication or a cancer risk indication based on the intensity data and reference data.

The intensity data may be related to image data. It may be possible to generate an image of the breast using the image data. The intensity data may be image pixel data or image voxel data.

The method may further comprise determining a parameter value based on the intensity data; and determining a density indication or a cancer risk indication based on a comparison between the parameter value and the reference data.

The intensity data may comprise a set of plural intensity values determined based on the intensity (or magnitude) of the received microwave signals. The intensity values may be image pixel or image voxel intensity values.

Thee intensity data may comprise histogram data representing a frequency density distribution curve across a range of intensity values, the intensity values being determined based on the intensity (or magnitude) of the received microwave signals. The intensity values may be image pixel or image voxel intensity values. The histogram data may be image histogram data. The method may further comprise determining the parameter value based on the histogram data.

The method may comprise determining the parameter value based on an area under at least a part of the curve that is represented by the histogram data. The method may comprise determining the parameter value based on a sum of elements with respect to at least a part of the curve that is represented by the histogram data.

The method may further comprise determining the parameter value for only a part of the frequency density distribution curve having intensity values falling within a range within the highest 50%, 45%, 40%, 35%, 30%, 25%, 20%, 15%, 10% or 5% of intensity values in the overall range of intensity values in the set of plural intensity values.

The reference data may comprise one or more reference values.

For a better understanding of the invention, and to show more clearly how it may be carried into effect, reference will now be made, by way of example, to the accompanying drawings, in which:-

Figure 1 is a system diagram of a medical imaging system according to an embodiment of the invention;

Figure 2 is a schematic view of the medical imaging system;

Figure 3 is a flowchart depicting a sampling method;

Figure 4 shows a flowchart illustrating the various processing steps of the present invention;

Figure 5 is a schematic illustration of a histogram of intensity data for a three-dimensional array of voxel data elements; and

Figure 6 schematically illustrates how results may be classified.

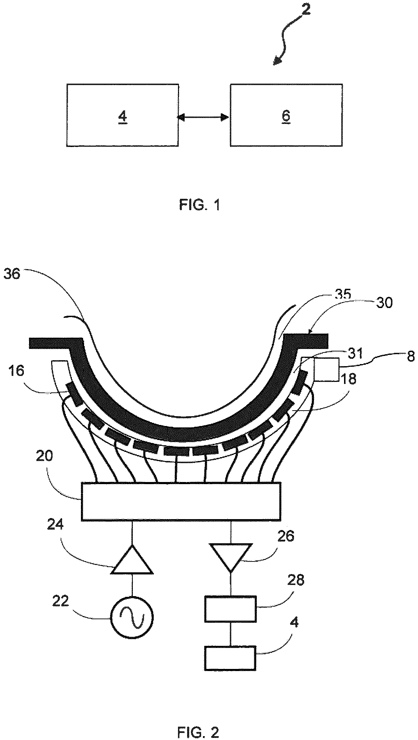

**Figure 1** shows a medical imaging system 2 according to an embodiment of the invention. The medical imaging system generally comprises a processor 4 and a microwave antenna array 6 in communication with the processor 4.

As shown in **Figure 2,** the antenna array 6 comprises a plurality N of antennas 16 which are arranged over the surface of, or within, a shell substrate 18. The shell 18 has a curved profile as shown. In particular, the shell 18 is part or hemi-spherical and is configured to approximate the shape of a breast. The antennas 16 are arranged over the shell 18 such that they all point to a common focal point.

The antennas 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4.

The switching matrix 20 selectively couples each of the antennas 16 to either the transmit path or the receive path.

The antenna array 6 is operated in a multi-static fashion. Specifically, the switching matrix 20 is controlled so as to connect one of the antennas 16 to the transmit path and the remaining antennas 16 to the receive path. The signal generator 22 generates a stepped frequency continuous wave (CW) signal which is amplified by the amplifier 24 and then transmitted by the antenna 16 connected to the transmit path. The stepped frequency continuous wave signal is a sequential series of pulses of continuous wave energy, where each pulse has its frequency stepped up across a range of frequencies, typically within the 3-8 GHz range. The other antennas 16 receive the transmitted signal and the received signal is detected and then recorded by the processor 4.

As shown in Figure 2, the shell 18 receives a cup 30. The cup 30 has a complementary shape to the shell 18 such that if fits snugly within the shell 18. A layer of coupling fluid (dielectric constant controlled fluid) may be inserted in the gap 31 between the shell 18 and the cup 30 so as to improve the coupling between the antennas 16 and the cup 30 in order to minimise signal loss and thus improve transmission of the microwave signal.

An actuator (not shown), such as a motor, may be connected to the microwave antenna array 6. The actuator is configured to move the microwave antenna array 6 relative to the cup 30 which remains stationary against the breast 36. Specifically, the actuator causes the microwave antenna array 6 to rotate relative to the cup 30 about the breast 36. The microwave antenna array 6 rotates about the center of the shell 18 (i.e. its axis of symmetry). This may be enabled by a threaded engagement between the cup 30 and the shell 18. In particular, the outside of the cup 30 and the inside of the shell 18 may have threaded portions which engage to allow the shell 18 and the antenna array 6 to be rotated relative to the cup 30. This may also allow the cup 30 to be quickly and easily removed so as to enable the coupling fluid to be replaced.

The antennas 16 may be as described in WO 2009/060181. In particular, the antennas 16 may comprise a slot 16 formed in a conductive element, the slot having a rectangular external boundary defined by a substantially closed internal edge of the conductive element. A microstrip feed line may be spaced from the conductive element by a dielectric substrate with the distal end of the line positioned at the geometric centre of the slot.

In use, a patient lies in a prone position such that their breast 36 sits in the cup 30. A layer of coupling fluid may also be provided in the gap 35 between the cup 30 and the breast 36 in order to improve coupling between the antennas 16 and the breast 36.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

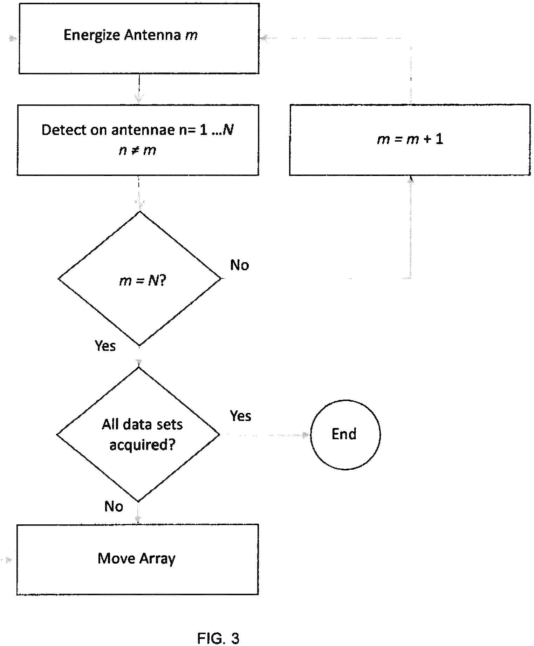

**Figure 3** shows a flowchart of a data acquisition method. As shown, the switching matrix 20 connects an antenna m of the N antennas to the transmit path. All other antennas 16 (n = 1...N, n≠m) are connected to the receive path. The antenna 16 connected to the transmit path illuminates the breast 36 with the microwave signal. The signal is scattered by the breast tissue and the scattered signal is received at each of the non-transmitting antennas 16 where it is detected (possibly in a time-sharing arrangement) and recorded. If m≠N, the switching matrix 20 steps to the next antenna 16 (m = m + 1) to be connected to the transmit path. This is repeated until all antennas 16 have been connected to the transmit path.

If additional data sets are required, then the actuator is used to rotate the array 6 relative to the breast 36 while retaining the breast in position. The acquisition process is then repeated with the antenna array 6 in the new configuration.

The processor 4 may record the relative difference between the measured phase and amplitude of the transmitted signal as compared to the phase and amplitude of the scattered signal, recorded as a complex number (having real and imaginary parts).

The signal detected at each antenna 16 is affected by scattering arising from objects within the imaged volume (i.e. the breast 36). In particular, tumours can generate significant reflections as they exhibit much higher dielectric properties than adipose and connective tissues due to their significant water content and so can be identified in the acquired data.

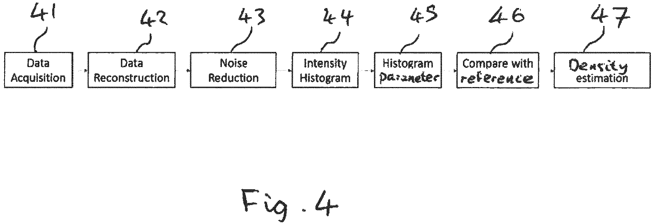

**Figure 4** shows a flowchart illustrating the various processing steps of the present invention.

The method begins at a data acquisition step 41. This step corresponds, in this arrangement, to the acquisition method described with respect to Figure 3.

The acquired data may be used at a data reconstruction step 42 to construct an image of the internal structure of the breast. The Data reconstruction may be performed using Phased Array (frequency domain), Delay and Sum (DAS - time domain) techniques or any other suitable technique, such as 3D Fourier Transformation, Back Projection, etc. From this, the processor 4 is able to identify (possibly, with additional user input or confirmation) a region of interest (if present) in which a possible tumour or other pathology may exist.

The reconstructed data may be subject to some calibration in order to remove components of the signals resulting from background noise, at step 43. For example, a set of measurements may be taken while the imaging volume (e.g. the interior of the cup 30) is filled with a fluid medium matching the dielectric permittivity of the cup 30 and the layer of dielectric constant controlled fluid in gap 31. This signal can then be corrected for in (e.g. subtracted from) the reconstructed data to calibrate and remove components of the signals which do not relate to the surface of the object (as reflections off the surface of the matching medium will be small or non-existent).

In the arrangement of Figure 4, it will be appreciated that at this point the reconstructed image will be in the form of an array of voxels corresponding to respective volumes within the breast, wherein each voxel has an intensity value that is indicative of the internal structure of the breast in the region in question.

At step 44, the processor generates intensity data that includes a set of intensity values for a three-dimensional data array of voxel elements and histogram data representing the voxel frequency density distribution for the intensity values. This may be done based on the reconstructed data representing an image of the internal structure of the breast. In such an arrangement, the processor will parse through the reconstructed data and determine, for each intensity value in the entire range of intensity values in the data, the number of voxels having the intensity value in question. The voxel frequency density can then be plotted against the corresponding intensity values (or "bins") to give a voxel frequency density distribution across the entire range of intensity values in the reconstructed data.

Once histogram data has been generated at step 44, the processor proceeds to step 45, where a parameter value is derived from the intensity values and the histogram data. In particular, the processor may be configured to determine the parameter value by calculating an area under the voxel frequency density curve.

In this example arrangement, the processor is configured to determine the parameter value for only the voxels having intensity values falling within a range corresponding to the highest 35% of intensity values for the overall image. However, it should be appreciated that in other arrangements different ranges may be selected.

For example, the highest 5%, 10%, 15%, 20%, 25%, 30%, 35%, 40%, 45%, 50% or 55% could be used (or any value in between, or any higher value).

As described above, the area under a histogram curve with N bins, and between bins *NI* and *N (NI<N),* may be determined according to the following equation:

$$AC = \frac{\sum_{i=Nl}^{N} n(i)}{C(N) - C(Nl)}$$

Where:

n(i) = Number of elements in bin i
C(N) = Intensity value at center of bin N
C(N1) = Intensity value at center of bin N1

After a value for the area under the highest 35% of the histogram curve has been determined, the processor compares the area parameter value against a predetermined reference value at step 46 of Figure 4. In this arrangement, the comparison includes determining whether the area value is above or below the pre-determined reference value.

If it is determined at step 46 that the area value is below the pre-determined reference value, the breast is characterised as being comprised mainly of dense tissue, at step 47, and the processor provides an indication of such. On the other hand, if it is determined at step 46 that the area value is above the pre-determined reference value, the breast is characterised as being comprised mainly of fatty tissue, at step 47, and the processor provides an indication of such.

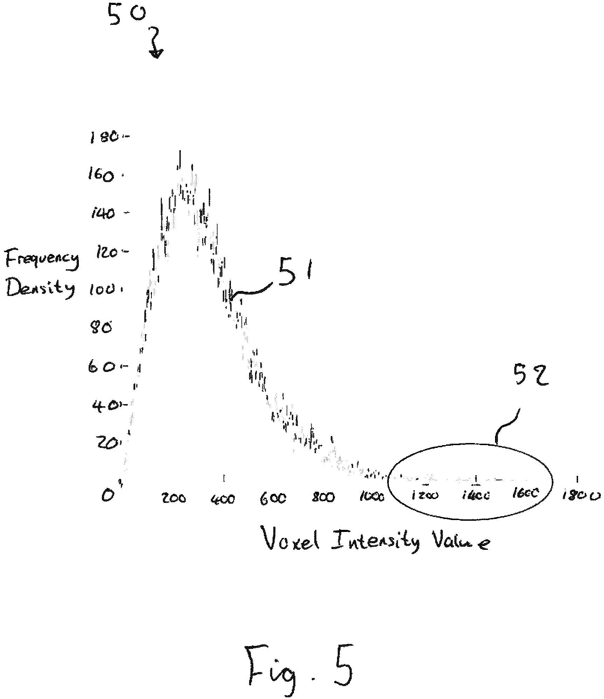

**Figure 5** is a schematic illustration of a histogram 50 of intensity data for a three-dimensional array of voxel elements. As can be seen, the histogram 50 shows a curve 51 of voxel frequency density across the entire range of intensity values in the reconstructed data.

Figure 5 also shows a region of interest in the voxel frequency density curve corresponding to the highest 35% of intensity values for the overall image, which will be the subject of the parameter value determination.

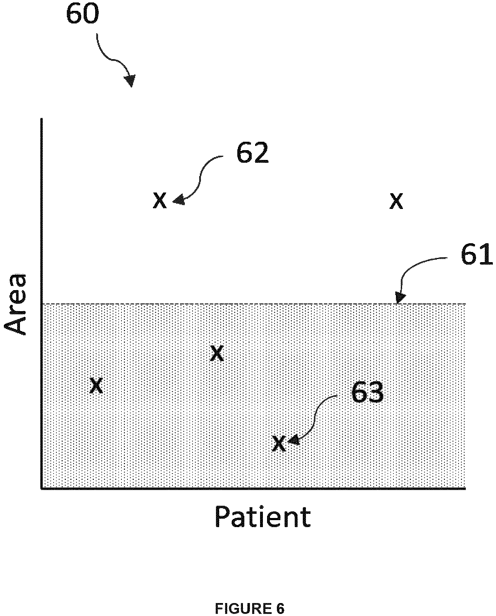

**Figure 6** is an example plot 60 showing the results of performing the method of the present invention for five cases, each represented by an x on the plot. If the determined area value for a given case is less than the pre-determined reference value (i.e. below line 61 in Figure 6), the sample for that case will be characterised as being dense. An example of a patient falling into this category is given by the point labelled 63. On the other hand, if the determined area value for a given case is greater than the pre-determined reference value (i.e. above line 65 in Figure 6), the sample for that case will be characterised as being fatty. An example of a patient falling into this category is given by the point labelled 62.

As mentioned above, it may be desirable to tailor the method of the present invention to breasts of different sizes.

In an arrangement, the present invention was used for a pool of cases that were pre-characterised according to size into three different classifications, small, medium and large, and for which a single reference value was determined in order to characterise the cases as being either dense or not.

In this arrangement, the present invention was used for a pool of 114 cases which were characterised beforehand as being large (i.e. falling within the large classification described above) and either fatty or dense. Table 1 shows the known distribution between fatty and dense tissue for the sample cases, determined from another imaging technique of known accuracy.

**Table 1 -** showing the known distribution between fatty and dense tissue for 114 sample cases precharacterised as being large.

| Size | Fatty | Dense | Total |
|------|-------|-------|-------|
| L | 50 | 64 | 114 |

The method of the present invention was then performed for each case in a manner as described above with respect to Figure 6, and by determining a parameter value corresponding to an area under a histogram curve.

It was found that for symptomatic and non-symptomatic breasts, 70% (80/114) of the cases were correctly classified as being either fatty or dense when using the area under the curve of the last 35 % of intensity values of the histogram. For symptomatic and non-symptomatic breasts, it was found that 82% (41/50) of the cases were correctly classified as being either fatty or dense when using the area under the curve of the last 35% of intensity values of the histogram.

Although the derivation of the density information has been described above in the detailed description the context of the medical imaging system 2, it will be appreciated that the density information may be generated from the raw data without generating an image of the breast 36. In fact, the density information may be provided separately in a standalone test where there is no cause for scanning the breast 36 for suspicious lesions. The system may therefore be embodied as a density measurement apparatus or meter which may or may not have the capability of imaging the breast. The present invention may serve as a useful pre-screening tool to identify the patient's risk score based on the breast density. This could be used to determine the screening frequency for the patient or other preventative measures based on the density assessment.

It will be appreciated that where only density information is required and a separate image of the breast for diagnostic purposes is not required, the system may be simplified from that described above. In particular, the system may use a single antenna which serves to transmit and receive the microwave signal or a pair of antennas which transmit and receive.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

The invention is not limited to the embodiments described herein, and may be modified or adapted without departing from the scope of the present invention.

**Claims**

1. A breast density meter for measuring the density of breast tissue comprising:

   at least one microwave antenna configured to transmit microwave signals so as to illuminate a breast of a patient and to receive the microwave signals following scattering within the breast; and
   a processor configured to generate intensity data based on the received microwave signals and determine a density indication or a cancer risk indication based on the intensity data and reference data.

2. A breast density meter as claimed in claim 1, wherein the processor is configured to:

   determine a parameter value based on the intensity data; and
   determine a density indication or a cancer risk indication based on a comparison between the parameter value and the reference data.

3. A breast density meter as claimed in claim 2, wherein the intensity data comprises a set of plural intensity values determined based on the magnitude of the received microwave signals.

4. A breast density meter as claimed in any preceding claim, wherein:

   the intensity data comprises histogram data representing a frequency density distribution curve across a range of intensity values, the intensity values being determined based on the magnitude of the received microwave signals; and
   the processor is configured to determine the parameter value based on the histogram data.

5. A breast density meter as claimed in claim 4, wherein the processor is configured to determine the parameter value based on an area under at least a part of the curve that is represented by the histogram data.

6. A breast density meter as claimed in claim 4, wherein the processor is configured to determine the parameter value based on a sum of elements with respect to at least a part of the curve that is represented by the histogram data.

7. A breast density meter as claimed in any preceding claim, wherein the intensity data comprises image voxel data.

8. A breast density meter as claimed in any preceding claim, wherein the reference data comprises one or more reference values.

9. A method for measuring the density of breast tissue comprising:

   illuminating a breast of a patient with microwave signals emitted by at least one microwave antenna;
   receiving the microwave signals following scattering within the breast at said at least one microwave antenna;
   generating intensity data based on the received microwave signals; and
   determining a density indication or a cancer risk indication based on the intensity data and reference data.

10. A method as claimed in claim 9, further comprising:

    determining a parameter value based on the intensity data; and
    determining a density indication or a cancer risk indication based on a comparison between the parameter value and the reference data.

11. A method as claimed in claim 10, wherein the intensity data comprises a set of plural intensity values determined based on the magnitude of the received microwave signals.

12. A method as claimed in claim 11, wherein:

    the intensity data comprises histogram data representing a frequency density distribution curve across a range of intensity values, the intensity values being determined based on the magnitude of the received microwave signals; and
    the method further comprises determining the parameter value based on the histogram data.

13. A method as claimed in claim 12, wherein the method comprises determining the parameter value based on an area under at least a part of the curve that is represented by the histogram data.

14. A method as claimed in claim 12, wherein the method comprises determining the parameter value based on a sum of elements with respect to at least a part of the curve that is represented by the histogram data.

15. A method as claimed in any of claims 9-14, wherein the intensity data comprises image voxel data.

FIG. 1

FIG. 2

Energize Antenna *m*

Detect on antennae n= 1 ...*N*
$n \neq m$

*m = N?* — No

Yes

$m = m + 1$

All data sets acquired? — Yes → End

No

Move Array

FIG. 3

Fig. 4

Fig. 5

FIGURE 6

Europäisches
Patentamt

European
Patent Office

Office européen
des brevets

# EUROPEAN SEARCH REPORT

Application Number

EP 17 19 5569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/058606 A1 (DAVIS SHAKTI K [US] ET AL) 16 March 2006 (2006-03-16) * figures 2, 3 * * paragraphs [0039], [0043], [0072], [0080] * | 1-15 | INV. A61B5/00 A61B5/05 |
| X | WO 2014/124304 A1 (TRUCTEES OF DARTMOUTH COLLEGE [US]) 14 August 2014 (2014-08-14) * figures 1, 4 * * paragraphs [0019], [0020], [0034] * | 1-15 | |
| X | US 2009/024026 A9 (SIMPKIN RAY A [NZ]) 22 January 2009 (2009-01-22) * figures 4, 7a, 7b * * paragraphs [0079], [0085], [0090], [0129], [0142] * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 25 January 2018 | Almeida, Mariana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 17 19 5569

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2018

| Patent document cited in search report | | | Publication date | Patent family member(s) | | | Publication date |
|---|---|---|---|---|---|---|---|
| US 2006058606 | A1 | | 16-03-2006 | NONE | | | |
| WO 2014124304 | A1 | | 14-08-2014 | US | 2015371380 | A1 | 24-12-2015 |
| | | | | WO | 2014124304 | A1 | 14-08-2014 |
| US 2009024026 | A9 | | 22-01-2009 | EP | 1788946 | A1 | 30-05-2007 |
| | | | | US | 2007060816 | A1 | 15-03-2007 |
| | | | | WO | 2006028396 | A1 | 16-03-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009060181 A